# EUROPEAN PATENT SPECIFICATION

(11) **EP 4 202 050 B1**
(45) Date of publication and mention of the grant of the patent: **02.07.2025**
(21) Application number: 21216448.7
(22) Date of filing: 21.12.2021
(51) Int. Cl.: C12P 7/18

(54) **METHOD FOR THE BIOTECHNOLOGICAL PRODUCTION OF ERYTHRITOL**
VERFAHREN ZUR BIOTECHNOLOGISCHEN HERSTELLUNG VON ERYTHRITOL
PROCÉDÉ DE LA PRODUCTION BIOTECHNOLOGIQUE D'ÉRYTHRITOL

(43) Date of publication of application: 28.06.2023
(73) Proprietor: Conzil Estate GmbH, 78351 Bodman-Ludwigshafen (DE)
(72) Inventor: MACH-AIGNER, Astrid, 1060 Vienna (AT); MACH, Robert, 1060 Vienna (AT); MASI, Audrey, 1060 Vienna (AT); DAZA SERNA, Laura Vanessa, 1060 Vienna (AT); FRIEDL, Anton, 1060 Vienna (AT); STARK, Georg, 1060 Vienna (AT)
(74) Representative: Ulrich, Thomas Franz

(56) References cited:
- EP-A2- 0 327 016
- WO-A1-2012/041894
- WO-A1-2013/096693
- LEE K J ET AL: "Optimized conditions for high erythritol production by Penicillium sp. KJ-UV29, mutant of Penicillium sp. KJ81", BIOTECHNOLOGY AND BIOPROCESS ENGINEERING, KOREAN SOCIETY FOR BIOTECHNOLOGY AND BIOENGINEERING, SEOUL, KR, vol. 8, no. 3, 1 June 2003 (2003-06-01), pages 173 - 178, XP008086830, ISSN: 1226-8372
- JOVANOVIC BIRGIT ET AL: "Erythritol production on wheat straw using Trichoderma reesei", AMB EXPRESS, vol. 4, no. 1, 1 December 2014 (2014-12-01), pages 34, XP55927743, Retrieved from the Internet <URL:https://amb-express.springeropen.com/track/pdf/10.1186/s13568-014-0034-y.pdf> DOI: 10.1186/s13568-014-0034-y
- DAVID HELGA ET AL: "CreA influences the metabolic fluxes of Aspergillus nidulans during growth on glucose and xylose", MICROBIOLOGY, vol. 151, no. 7, 1 July 2005 (2005-07-01), Reading, pages 2209 - 2221, XP055927826, ISSN: 1350-0872, Retrieved from the Internet <URL:https://www.microbiologyresearch.org/docserver/fulltext/micro/151/7/mic1512209.pdf?expires=1654247773&id=id&accname=guest&checksum=8BBE37D4EFFBE5F5336B066B0EF7EC08> DOI: 10.1099/mic.0.27787-0
- HEE-JUNG MOON ET AL: "Biotechnological production of erythritol and its applications", APPLIED MICROBIOLOGY AND BIOTECHNOLOGY, SPRINGER, BERLIN, DE, vol. 86, no. 4, 26 February 2010 (2010-02-26), pages 1017 - 1025, XP019800001, ISSN: 1432-0614
- MASI AUDREY ET AL: "Exploration of Trichoderma reesei as an alternative host for erythritol production", BIOTECHNOLOGY FOR BIOFUELS AND BIOPRODUCTS, vol. 17, 90, 27 June 2024 (2024-06-27), pages 1 - 19, XP093224503, ISSN: 2731-3654, Retrieved from the Internet <URL:https://link.springer.com/article/10.1186/s13068-024-02537-x/fulltext.html> DOI: 10.1186/s13068-024-02537-x

## Description

The present invention pertains to a method for the biotechnological production of erythritol, in particular a method for the biotechnological production of erythritol by cultivating at least one saprotroph selected from the genera *Hypocrea* and *Trichoderma* in a culture medium comprising a nitrogen source and 60 to 100 g/L of a monomeric or oligomeric C-6 sugar or a mixture thereof, wherein the C-6 sugar is selected from the group consisting of glucose and fructose.

In the recent years people's lifestyle and the growing consumption of food products with high sugar content has resulted in a tremendous rise of blood glucose related diseases and disorders, such as diabetes mellitus type 2 (DMT2). Nowadays, a low-glycaemic nutrition and the avoidance of excessive peaks in blood glucose level is considered to reduce the risk for developing certain chronic diseases and to be beneficial for maintenance and improvement of health and for the treatment and/or prevention of a large number of blood glucose related diseases and disorders.

Erythritol is a naturally occurring four-carbon sugar alcohol gaining increasing importance in the food industry due to its specific properties and its manifold fields of application. It can be found in several fruits, such as pears, grapes and melons, mushrooms, alcoholic drinks (beer, wine, sake) and fermented food products, such as soy sauce and miso bean paste, but naturally also occurs in biofluids of humans and animals such as eye lens tissue, serum, plasma, fetal fluid and urine. Due to its small molecular weight, erythritol is easily absorbed already in the upper intestine and therefore causes less digestive distress than other sugar alcohols used in the food industry. The majority of ingested erythritol is not metabolized in the human body and is excreted unmodified into the urine without changing blood glucose and insulin levels (Regnat et al., Erythritol as sweetener-wherefrom and whereto?, 2018, Applied Microbiology and Biotechnology, Vol. 102). Furthermore, erythritol is non-cariogenic, thermally stable, crystalizes well and is less hygroscopic than sucrose. Due to the negative enthalpy of dissolution, the consumption of erythritol causes a cooling sensation in the oral cavity. A 10% (w/v) solution of erythritol has 60-80% of the sweetness of sucrose at the same concentration.

However, in contrast to other sugar alcohols, such as sorbitol, xylitol, mannitol, lactitol, and maltitol, which are well-established as sugar alternatives for many years, so far erythritol cannot be chemically produced in a commercially worthwhile way. The production of erythritol from dialdehyde starch using a nickel catalyst at high temperatures results in unsatisfying low yields (Moon et al. Biotechnological production of erythritol and its applications, 2010, Appl. Microbiol. Biotechnol., Vol. 86).

In yeast and fungus species, erythritol is produced via the so-called pentose phosphate pathway. It is synthesized from D-erythrose-4-phosphate through dephosphorylation and subsequent reduction of erythrose. Based thereon, the suitability of osmophilic yeast, such as *Aureobasidium* sp., *Trichosporonoides* sp. and *Candida magnoliae,* for the biotechnological production of erythritol has been investigated in several studies (Ishizuka et al., Breeding of a mutant of Aureobasidium sp. with high erythritol production, 1989, J. Ferm. Bioeng., Vol. 68(5); US4939091A; US5962287 A; Oh et al., Increased erythritol production in fedbatch cultures of Torula sp. by controlling glucose concentration, 2001, JIM&B, Vol. 26; Koh et al., Scale-up of erythritol production by an osmophilic mutant of Candida magnoliae, 2003, Biotechnol. Lett., Vol. 25; Ryu et al., Optimization of erythritol production by Candida magnoliae in fed-batch culture, 2000, JIM&B, Vol. 25). More recent studies examined the potential of filamentous fungi to produce erythritol (Jovanovic et al., 2013). Lee et al. (Biotechnology and Bioprocess Engineering, vol. 8, no. 3, 2003) describes the generation and selection of mutant strains derived from the erythritol producer strain *Penicillium sp.* KJ81 as well as the optimization of the conditions used for cultivation with the purpose of improving erythritol productivity. Jovanovic et al. (Amb Express, vol. 4, no. 1, 2014) discloses the production of erythritol from wheat straw using *Trichoderma reesei.* WO 2013/096693 A1 teaches processing of biomass feedstock materials, in particular cellulosic and lignocellulosic materials, using microorganisms to produce erythritol. WO 2012/041894 A1 discloses the use of erythrose reductase which can be derived from *Hypocrea, Gibberella, Aspergillus* and *Penicillium* for the conversion of erythrose to erythritol. David et al. (Microbiology, vol. 151, no. 7, 2005) investigated the metabolic fluxes and the erythritol production of *Aspergillus nidulans* using CreA mutant strains. EP 0 327 016 B1 is directed to a method for the isolation of highly pure erythritol produced by the bacterial strain *Aureobasidium* SN-G42. However, the yields of erythritol obtained from the different strains was unsatisfactory for industrial scale production.

Accordingly, there is a need for a biotechnological method for the production of erythritol with increased yield. The present invention overcomes the disadvantages of the methods in the prior art by the subject-matter of the independent claims, in particular by the method for the production of erythritol according to the present invention.

The present invention in particular pertains to a method for the production of erythritol, comprising the steps:
a) cultivating at least one saprotroph selected from the genera *Hypocrea* and *Trichoderma* in a culture medium comprising a carbon source in a concentration of 60 to 100 g/L and a nitrogen source, so as to obtain erythritol in the culture medium, and
b) recovering erythritol from the culture medium,
wherein the carbon source is a monomeric or oligomeric C-6 sugar or a mixture thereof, wherein the C-6 sugar is selected from the group consisting of glucose and fructose.

In a preferred embodiment of the present invention, the culture medium comprises the carbon source in a concentration of at least 70 g/L.

In a further preferred embodiment of the present invention, the culture medium comprises the carbon source in a concentration of at most 95 g/L, preferably at most 90 g/L.

According to a preferred embodiment of the present invention, the culture medium comprises the carbon source in a concentration of 65 to 95 g/L, preferably 70 to 90 g/L.

In a further preferred embodiment of the present invention, the culture medium comprises the nitrogen source in a concentration of at least 50 mM, preferably at least 55 mM, preferably at least 60 mM, preferably at least 65 mM, preferably at least 70 mM.

Preferably, the culture medium comprises the nitrogen source in a concentration of at most 150 mM, preferably at most 140 mM, preferably at most 130 mM, preferably at most 120 mM, preferably at most 110 mM, preferably at most 100 mM.

Particularly preferred, the culture medium comprises the nitrogen source in a concentration of 50 to 140 mM, preferably 55 to 130 mM, preferably 60 to 120 mM, preferably 65 to 110 mM, preferably 70 to 100 mM.

According to preferred embodiment of the present invention, the culture medium is a synthetic medium. Preferably, the culture medium is not a synthetic medium.

In a further preferred embodiment of the present invention, the culture medium comprises a hydrolysate obtained by hydrothermal treatment of a cellulose-, and/or starch-comprising raw material.

Particularly preferred, the culture medium comprises a lignocellulose-comprising hydrolysate.

In a further preferred embodiment of the present invention, the hydrolysate, preferably the hydrolysate obtained by hydrothermal treatment of a cellulose-, and/or starch-comprising raw material, in particular the lignocellulose-comprising hydrolysate, is derived from agro-industrial residues.

According to preferred embodiment of the present invention, the culture medium comprises a hydrolysate of straw, in particular a hydrolysate of wheat straw. In a further preferred embodiment, the culture medium comprises a hydrolysate of wheat bran. Preferably, the culture medium comprises a hydrolysate of potato pulp.

According to the present invention, the carbon source is a monomeric or oligomeric C-6 sugar or a mixture thereof, wherein the monomeric or oligomeric C-6 sugar is selected from glucose and fructose. According to a preferred embodiment, the carbon source is a mixture of monomeric and oligomeric C-6 sugars selected from glucose and fructose.

In a further preferred embodiment, the carbon source is glucose.

According to a preferred option, the C-5 sugar content in the culture medium amounts to at least 5 %, preferably at least 10 %, preferably at least 15 %, preferably at least 20 %, preferably at least 25 %, preferably at least 30 %, preferably at least 40 %, preferably at least 50 %, preferably at least 60 %, preferably at least 70 %, preferably at least 80 %, preferably at least 85 %, preferably at least 90 %, preferably at least 95 % (based on total carbohydrates in the culture medium).

Preferably, the C-6 sugar content in the culture medium amounts to at least 10 %, preferably at least 15 %, preferably at least 20 %, preferably at least 25 %, preferably at least 30 %, preferably at least 40 %, preferably at least 50 %, preferably at least 60 %, preferably at least 70 %, preferably at least 80 %, preferably at least 85 %, preferably at least 90 %, preferably at least 95 % (based on total carbohydrates in the culture medium).

According to a preferred option, the glucose content in the culture medium amounts to at least 10 %, preferably at least 15 %, preferably at least 20 %, preferably at least 25 %, preferably at least 30 %, preferably at least 40 %, preferably at least 50 %, preferably at least 60 %, preferably at least 70 %, preferably at least 80 %, preferably at least 85 %, preferably at least 90 %, preferably at least 95 % (based on total carbohydrates in the culture medium).

According to another preferred option, the xylose content in the culture medium amounts to at least 10 %, preferably at least 15 %, preferably at least 20 %, preferably at least 25 %, preferably at least 30 %, preferably at least 40 %, preferably at least 50 %, preferably at least 60 %, preferably at least 70 %, preferably at least 80 %, preferably at least 85 %, preferably at least 90 %, preferably at least 95 % (based on total carbohydrates in the culture medium).

Preferably, the content of monomeric C-5 and C-6 sugars in the culture medium amounts to at least 1 %, preferably at least 2 %, preferably at least 3 %, preferably 4 %, preferably at least 5 %, preferably at least 6 %, preferably at least 7 %, preferably at least 8 %, preferably at least 9 %, preferably at least 10 %, preferably at least 15 %, preferably at least 20 %, preferably at least 30 %, preferably at least 40 %, preferably at least 50 %, preferably at least 60 %, preferably at least 70 %, preferably at least 80 %, preferably at least 85 %, preferably at least 90 %, preferably at least 95 %, preferably at least 96 %, preferably at least 97 %, preferably at least 98 %, preferably at least 99 % (based on total carbohydrates in the culture medium).

According to a preferred option, the content of oligomeric C-5 and C-6 sugars in the culture medium amounts to at least 10 %, preferably at least 20 %, preferably at least 30 %, preferably at least 40 %, preferably at least 50 %, preferably at least 60 %, preferably at least 70 %, preferably at least 75 %, preferably at least 80 %, preferably at least 85 %, preferably at least 90 %, preferably at least 95 %, preferably at least 96 %, preferably at least 97 %, preferably at least 98 %, preferably at least 99 % (based on total carbohydrates in the culture medium).

In a preferred embodiment of the present invention, the nitrogen source is urea.

According to the present invention, the at least one saprotroph is a filamentous fungus selected from the genera *Hypocrea* and *Trichoderma.*

In a preferred embodiment, the saprotroph is *Hypocrea jecorina (Trichoderma reesei).*

According a preferred option, the at least one saprotroph is a naturally occurring saprotroph, in particular is not a genetically modified saprotroph.

According to another preferred option, the at least one saprotroph is genetically modified.

In a particularly preferred option, the genetically modified saprotroph is *Hypocrea jecorina (Trichoderma reesei).* Preferably, the genetically modified saprotroph is based on the *Trichoderma reesei* strain QM6aΔ*tmus53.*

Preferably, the at least one genetically modified saprotroph comprises at least one gene encoding at least one membrane-bound alditol transporter, at least one gene encoding at least one erythrose reductase and at least one inactivated gene encoding mannitol 1-phosphate 5-dehydrogenase.

Preferably, the at least one gene of the genetically modified saprotroph encoding at least one membrane-bound alditol transporter is *fps1*, in particular codon-optimized *fps1.* Preferably, the at least one gene of the genetically modified saprotroph encoding at least one membrane-bound alditol transporter is *fps1* from *Saccharomyces cerevisiae,* in particular is codon-optimized *fps1* from *Saccharomyces cerevisiae.*

Preferably, the at least one gene of the genetically modified saprotroph encoding at least one membrane-bound alditol transporter comprises the nucleotide sequence of SEQ ID No. 1, in particular consists of the nucleotide sequence of SEQ ID No. 1.

Preferably, the at least one gene of the genetically modified saprotroph encoding at least one membrane-bound alditol transporter comprises a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 1. Preferably, the at least one gene of the genetically modified saprotroph encoding at least one membrane-bound alditol transporter consists of a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 1.

Preferably, the membrane-bound alditol transporter of the genetically modified saprotroph comprises the amino acid sequence of SEQ ID No. 2, in particular consists of the amino acid sequence of SEQ ID No. 2.

Preferably, the membrane-bound alditol transporter of the genetically modified saprotroph comprises an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 2. According to another preferred option, the membrane-bound alditol transporter of the genetically modified saprotroph consists of an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 2.

According to another preferred option, the membrane-bound alditol transporter of the genetically modified saprotroph comprises an amino acid sequence as defined in SEQ ID No. 2, in which one, preferably two, preferably three, preferably five, preferably six, preferably seven, preferably eight, preferably nine, preferably ten, amino acids are exchanged by other naturally occurring amino acids. Particularly preferred, the membrane-bound alditol transporter of the genetically modified saprotroph comprises an amino acid sequence as defined in SEQ ID No. 2, in which at most ten, preferably at most nine, preferably at most eight, preferably at most seven, preferably at most six, preferably at most five, preferably at most four, preferably at most three, preferably at most two, preferably at most one, amino acids are exchanged by other naturally occurring amino acids.

Preferably, the at least one gene of the genetically modified saprotroph encoding at least one erythrose reductase is *err1,* in particular is codon-optimized *err1.* Preferably, the at least one gene of the genetically modified saprotroph encoding at least one erythrose reductase is *err1,* in particular is codon-optimized *err1,* from *Trichoderma reesei*, *Aspergillus niger* or *Fusarium graminearum.*

Preferably, the at least one gene of the genetically modified saprotroph encoding at least one erythrose reductase comprises the nucleotide sequence of SEQ ID No. 3, in particular consist of the nucleotide sequence of SEQ ID No. 3.

Preferably, the at least one gene of the genetically modified saprotroph encoding at least one erythrose reductase comprises a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 3 According to another preferred option, the at least one gene of the genetically modified saprotroph encoding at least one gene encoding at least one erythrose reductase consists of a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 3.

Preferably, the erythrose reductase of the genetically modified saprotroph comprises the amino acid sequence of SEQ ID No. 4, in particular consists of the amino acid sequence of SEQ ID No. 4.

Preferably, the erythrose reductase of the genetically modified saprotroph comprises an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 4. Particularly preferred, the erythrose reductase of the genetically modified saprotroph consists of an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 4.

Preferably, the erythrose reductase of the genetically modified saprotroph comprises an amino acid sequence as defined in SEQ ID No. 4, in which one, preferably two, preferably three, preferably five, preferably six, preferably seven, preferably eight, preferably nine, preferably ten, amino acids are exchanged by other naturally occurring amino acids. Preferably, the erythrose reductase of the genetically modified saprotroph comprises an amino acid sequence as defined in SEQ ID No. 4, in which at most ten, preferably at most nine, preferably at most eight, preferably at most seven, preferably at most six, preferably at most five, preferably at most four, preferably at most three, preferably at most two, preferably at most one, amino acids are exchanged by other naturally occurring amino acids.

According to a preferred option, the at least one inactivated gene encoding mannitol 1-phosphate 5-dehydrogenase of the genetically modified saprotroph is *mpdh.* Preferably, the at least one gene encoding mannitol 1-phosphate 5-dehydrogenase of the genetically modified saprotroph comprised the nucleotide sequence of SEQ ID No. 9 before inactivation, in particular consisted of the nucleotide sequence of SEQ ID No. 9 before inactivation.

Preferably, the at least one gene encoding mannitol 1-phosphate 5-dehydrogenase of the genetically modified saprotroph comprised a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 9 before inactivation. According to another preferred option, the at least one gene encoding mannitol 1-phosphate 5-dehydrogenase of the genetically modified saprotroph consisted of a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 9 before inactivation.

Preferably, the mannitol 1-phosphate 5-dehydrogenase encoded by the nucleotide sequence of SEQ ID No. 9 before inactivation comprises the amino acid sequence of SEQ ID No. 10, in particular consists of the amino acid sequence of SEQ ID No. 10.

According to another preferred option, the mannitol 1-phosphate 5-dehydrogenase encoded by the nucleotide sequence of SEQ ID No. 9 before inactivation comprises an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 10. Particularly preferred, the mannitol 1-phosphate 5-dehydrogenase encoded by the nucleotide sequence of SEQ ID No. 9 before inactivation consists of an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 10.

Preferably, the mannitol 1-phosphate 5-dehydrogenase encoded by the nucleotide sequence of SEQ ID No. 9 before inactivation comprises an amino acid sequence as defined in SEQ ID No. 10, in which one, preferably two, preferably three, preferably five, preferably six, preferably seven, preferably eight, preferably nine, preferably ten, amino acids are exchanged by other naturally occurring amino acids. Preferably, the mannitol 1-phosphate 5-dehydrogenase encoded by the nucleotide sequence of SEQ ID No. 9 before inactivation comprises an amino acid sequence as defined in SEQ ID No. 10, in which at most ten, preferably at most nine, preferably at most eight, preferably at most seven, preferably at most six, preferably at most five, preferably at most four, preferably at most three, preferably at most two, preferably at most one, amino acids are exchanged by other naturally occurring amino acids.

According to a further preferred option, the at least one inactivated gene encoding mannitol 1-phosphate 5-dehydrogenase of the genetically modified saprotroph is deleted.

Preferably, the at least one inactivated gene encoding mannitol 1-phosphate 5-dehydrogenase of the genetically modified saprotroph is non-functional.

Particularly preferred, the at least one inactivated gene encoding mannitol 1-phosphate 5-dehydrogenase of the genetically modified saprotroph is inactivated by gene knock-out, in particular gene replacement. Preferably, the at least one inactivated gene encoding mannitol 1-phosphate 5-dehydrogenase of the genetically modified saprotroph is inactivated by gene replacement using a deletion cassette.

Preferably, the at least one gene encoding mannitol 1-phosphate 5-dehydrogenase of the genetically modified saprotroph is inactivated, in particular made non-functional, by genome editing, in particular by meganucleases, zinc-finger nucleases (ZFNs), transcription activator-like effector nucleases (TALENs) or by the clustered regularly interspaced short palindromic repeats (CRISPR) system.

Particularly preferred, the genetically modified saprotroph comprises at least one, preferably at least two, preferably at least three, preferably at least four, preferably at least five, further inactivated genes. Preferably, the at least one further inactivated gene of the genetically modified saprotroph is selected from the group consisting of a gene encoding phospho-2-dehydro-3-deoxyheptonate aldolase 1 (Dhaps-1), a gene encoding erythritol utilization factor (EUF), a gene encoding erythrulose kinase (EYK1), a gene encoding erythritol dehydrogenase (EYD1), a gene encoding erythritol isomerase 1 (EYI1) and a gene encoding erythritol isomerase 2 (EYI2).

Preferably, the at least one further inactivated gene of the genetically modified saprotroph is a gene encoding phospho-2-dehydro-3-deoxyheptonate aldolase 1 (Dhaps-1). Preferably, the at least one gene encoding phospho-2-dehydro-3-deoxyheptonate aldolase 1 (Dhaps1) of the genetically modified saprotroph comprised the nucleotide sequence of SEQ ID No. 11 before inactivation, in particular consisted of the nucleotide sequence of SEQ ID No. 11 before inactivation.

Preferably, the at least one gene encoding phospho-2-dehydro-3-deoxyheptonate aldolase 1 (Dhaps1) of the genetically modified saprotroph comprised a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 11 before inactivation. Further preferably, the at least one gene encoding phospho-2-dehydro-3-deoxyheptonate aldolase 1 (Dhaps1) of the genetically modified saprotroph consisted of a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 11 before inactivation.

Preferably, the phospho-2-dehydro-3-deoxyheptonate aldolase 1 (Dhaps1) encoded by the nucleotide sequence of SEQ ID No. 11 before inactivation comprises the amino acid sequence of SEQ ID No. 12, in particular consists of the amino acid sequence of SEQ ID No. 12.

Preferably, the phospho-2-dehydro-3-deoxyheptonate aldolase 1 (Dhaps1) encoded by the nucleotide sequence of SEQ ID No. 11 before inactivation comprises an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 12. Particularly preferred, the phospho-2-dehydro-3-deoxyheptonate aldolase 1 (Dhaps1) encoded by the nucleotide sequence of SEQ ID No. 11 before inactivation consists of an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 12.

Preferably, the phospho-2-dehydro-3-deoxyheptonate aldolase 1 (Dhaps1) encoded by the nucleotide sequence of SEQ ID No. 11 before inactivation comprises an amino acid sequence as defined in SEQ ID No. 12, in which one, preferably two, preferably three, preferably five, preferably six, preferably seven, preferably eight, preferably nine, preferably ten, amino acids are exchanged by other naturally occurring amino acids. Preferably, the phospho-2-dehydro-3-deoxyheptonate aldolase 1 (Dhaps1) encoded by the nucleotide sequence of SEQ ID No. 11 before inactivation comprises an amino acid sequence as defined in SEQ ID No. 12, in which at most ten, preferably at most nine, preferably at most eight, preferably at most seven, preferably at most six, preferably at most five, preferably at most four, preferably at most three, preferably at most two, preferably at most one, amino acids are exchanged by other naturally occurring amino acids.

According to another option, the at least one further inactivated gene of the genetically modified saprotroph is a gene encoding erythritol utilization factor (EUF). Preferably, the at least one gene encoding erythritol utilization factor (Eufl) of the genetically modified saprotroph comprised the nucleotide sequence of SEQ ID No. 13 before inactivation, in particular consisted of the nucleotide sequence of SEQ ID No. 13 before inactivation.

Preferably, the at least one gene encoding erythritol utilization factor (Eufl) of the genetically modified saprotroph comprised a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 13 before inactivation. Particularly preferred, the at least one gene encoding erythritol utilization factor (Eufl) of the genetically modified saprotroph consisted of a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 13 before inactivation.

Preferably, the erythritol utilization factor (Eufl) encoded by the nucleotide sequence of SEQ ID No. 13 before inactivation comprises the amino acid sequence of SEQ ID No. 14, in particular consists of the amino acid sequence of SEQ ID No. 14.

Preferably, the erythritol utilization factor (Eufl) encoded by the nucleotide sequence of SEQ ID No. 13 before inactivation comprises an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 14. Particularly preferred, the erythritol utilization factor (Eufl) encoded by the nucleotide sequence of SEQ ID No. 13 before inactivation consists of an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 14.

Preferably, the erythritol utilization factor (Eufl) encoded by the nucleotide sequence of SEQ ID No. 13 before inactivation comprises an amino acid sequence as defined in SEQ ID No. 14, in which one, preferably two, preferably three, preferably five, preferably six, preferably seven, preferably eight, preferably nine, preferably ten, amino acids are exchanged by other naturally occurring amino acids. Preferably, the erythritol utilization factor (Euf1) encoded by the nucleotide sequence of SEQ ID No. 13 before inactivation comprises an amino acid sequence as defined in SEQ ID No. 14, in which at most ten, preferably at most nine, preferably at most eight, preferably at most seven, preferably at most six, preferably at most five, preferably at most four, preferably at most three, preferably at most two, preferably at most one, amino acids are exchanged by other naturally occurring amino acids.

In a particularly preferred embodiment of the present invention, the at least one further inactivated gene of the genetically modified saprotroph is a gene encoding erythrulose kinase (EYK1). Preferably, the at least one gene encoding erythrulose kinase (Eyk1) of the genetically modified saprotroph comprised the nucleotide sequence of SEQ ID No. 15 before inactivation, in particular consisted of the nucleotide sequence of SEQ ID No. 15 before inactivation.

According to another preferred option, the at least one gene encoding erythrulose kinase (Eyk1) of the genetically modified saprotroph comprised a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 15 before inactivation. Particularly preferred, the at least one gene encoding erythrulose kinase (Eyk1) of the genetically modified saprotroph consisted of a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 15 before inactivation.

Preferably, the erythrulose kinase (Eyk1) encoded by the nucleotide sequence of SEQ ID No. 15 before inactivation comprises the amino acid sequence of SEQ ID No. 16, in particular consists of the amino acid sequence of SEQ ID No. 16.

Preferably, the erythrulose kinase (Eyk1) encoded by the nucleotide sequence of SEQ ID No. 15 before inactivation comprises an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 16. Particularly preferred, the erythrulose kinase (Eyk1) encoded by the nucleotide sequence of SEQ ID No. 15 before inactivation consists of an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 16.

Preferably, the erythrulose kinase (Eyk1) encoded by the nucleotide sequence of SEQ ID No. 15 before inactivation comprises an amino acid sequence as defined in SEQ ID No. 16, in which one, preferably two, preferably three, preferably five, preferably six, preferably seven, preferably eight, preferably nine, preferably ten, amino acids are exchanged by other naturally occurring amino acids. Preferably, the erythrulose kinase (Eyk1) encoded by the nucleotide sequence of SEQ ID No. 15 before inactivation comprises an amino acid sequence as defined in SEQ ID No. 16, in which at most ten, preferably at most nine, preferably at most eight, preferably at most seven, preferably at most six, preferably at most five, preferably at most four, preferably at most three, preferably at most two, preferably at most one, amino acids are exchanged by other naturally occurring amino acids.

Preferably, the at least one further inactivated gene of the genetically modified saprotroph is a gene encoding erythritol dehydrogenase (EYD1). Preferably, the at least one gene encoding erythritol dehydrogenase (Eyd1) of the genetically modified saprotroph comprised the nucleotide sequence of SEQ ID No. 17 before inactivation, in particular consisted of the nucleotide sequence of SEQ ID No. 17 before inactivation.

Preferably, the at least one gene encoding erythritol dehydrogenase (Eyd1) of the genetically modified saprotroph comprised a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 17 before inactivation. According to another preferred option, the at least one gene encoding erythritol dehydrogenase (Eyd1) of the genetically modified saprotroph consisted of a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 17 before inactivation.

Preferably, the erythritol dehydrogenase (Eyd1) encoded by the nucleotide sequence of SEQ ID No. 17 before inactivation comprises the amino acid sequence of SEQ ID No. 18, in particular consists of the amino acid sequence of SEQ ID No. 18.

Preferably, the erythritol dehydrogenase (Eyd1) encoded by the nucleotide sequence of SEQ ID No. 17 before inactivation comprises an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 18. Particularly preferred, the erythritol dehydrogenase (Eyd1) encoded by the nucleotide sequence of SEQ ID No. 17 before inactivation consists of an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 18.

Preferably, the erythritol dehydrogenase (Eyd1) encoded by the nucleotide sequence of SEQ ID No. 17 before inactivation comprises an amino acid sequence as defined in SEQ ID No. 18, in which one, preferably two, preferably three, preferably five, preferably six, preferably seven, preferably eight, preferably nine, preferably ten, amino acids are exchanged by other naturally occurring amino acids. Preferably, the erythritol dehydrogenase (Eyd1) encoded by the nucleotide sequence of SEQ ID No. 17 before inactivation comprises an amino acid sequence as defined in SEQ ID No. 18, in which at most ten, preferably at most nine, preferably at most eight, preferably at most seven, preferably at most six, preferably at most five, preferably at most four, preferably at most three, preferably at most two, preferably at most one, amino acids are exchanged by other naturally occurring amino acids.

Preferably, the at least one further inactivated gene of the genetically modified saprotroph is a gene encoding erythritol isomerase 1 (EYI1). Preferably, the at least one gene encoding erythritol isomerase 1 (Eyi1) of the genetically modified saprotroph comprised the nucleotide sequence of SEQ ID No. 19 before inactivation, in particular consisted of the nucleotide sequence of SEQ ID No. 19 before inactivation.

Particularly preferred, the at least one gene encoding erythritol isomerase 1 (Eyi1) of the genetically modified saprotroph comprised a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 19 before inactivation. According to another preferred option, the at least one gene encoding erythritol isomerase 1 (Eyi1) of the genetically modified saprotroph consisted of a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 19 before inactivation.

Preferably, the erythritol isomerase 1 (Eyi1) encoded by the nucleotide sequence of SEQ ID No. 19 before inactivation comprises the amino acid sequence of SEQ ID No. 20, in particular consists of the amino acid sequence of SEQ ID No. 20.

Preferably, the erythritol isomerase 1 (Eyi1) encoded by the nucleotide sequence of SEQ ID No. 19 before inactivation comprises an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 20. Particularly preferred, the erythritol isomerase 1 (Eyi1) encoded by the nucleotide sequence of SEQ ID No. 19 before inactivation consists of an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 20.

Preferably, the erythritol isomerase 1 (Eyi1) encoded by the nucleotide sequence of SEQ ID No. 19 before inactivation comprises an amino acid sequence as defined in SEQ ID No. 20, in which one, preferably two, preferably three, preferably five, preferably six, preferably seven, preferably eight, preferably nine, preferably ten, amino acids are exchanged by other naturally occurring amino acids. Preferably, the erythritol isomerase 1 (Eyi1) encoded by the nucleotide sequence of SEQ ID No. 19 before inactivation comprises an amino acid sequence as defined in SEQ ID No. 20, in which at most ten, preferably at most nine, preferably at most eight, preferably at most seven, preferably at most six, preferably at most five, preferably at most four, preferably at most three, preferably at most two, preferably at most one, amino acids are exchanged by other naturally occurring amino acids.

According to another preferred option, the at least one further inactivated gene of the genetically modified saprotroph is a gene encoding erythritol isomerase 2 (EYI2). Preferably, the at least one gene encoding erythritol isomerase 2 (Eyi2) of the genetically modified saprotroph comprised the nucleotide sequence of SEQ ID No. 21 before inactivation, in particular consisted of the nucleotide sequence of SEQ ID No. 21 before inactivation.

Preferably, the at least one gene encoding erythritol isomerase 2 (Eyi2) of the genetically modified saprotroph comprised a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 21 before inactivation. Preferably, the at least one gene encoding erythritol isomerase 2 (Eyi2) of the genetically modified saprotroph consisted of a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 21 before inactivation.

Preferably, the erythritol isomerase 2 (Eyi2) encoded by the nucleotide sequence of SEQ ID No. 21 before inactivation comprises the amino acid sequence of SEQ ID No. 22, in particular consists of the amino acid sequence of SEQ ID No. 22.

Preferably, the erythritol isomerase 2 (Eyi2) encoded by the nucleotide sequence of SEQ ID No. 21 before inactivation comprises an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 22. Particularly preferred, the erythritol isomerase 2 (Eyi2) encoded by the nucleotide sequence of SEQ ID No. 21 before inactivation consists of an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 22.

Preferably, the erythritol isomerase 2 (Eyi2) encoded by the nucleotide sequence of SEQ ID No. 21 before inactivation comprises an amino acid sequence as defined in SEQ ID No. 22, in which one, preferably two, preferably three, preferably five, preferably six, preferably seven, preferably eight, preferably nine, preferably ten, amino acids are exchanged by other naturally occurring amino acids. Preferably, the erythritol isomerase 2 (Eyi2) encoded by the nucleotide sequence of SEQ ID No. 21 before inactivation comprises an amino acid sequence as defined in SEQ ID No. 22, in which at most ten, preferably at most nine, preferably at most eight, preferably at most seven, preferably at most six, preferably at most five, preferably at most four, preferably at most three, preferably at most two, preferably at most one, amino acids are exchanged by other naturally occurring amino acids.

According to another preferred option, the at least one further inactivated gene selected from the group consisting of a gene encoding phospho-2-dehydro-3-deoxyheptonate aldolase 1 (Dhaps-1), a gene encoding erythritol utilization factor (EUF), a gene encoding erythrulose kinase (EYK1), a gene encoding erythritol dehydrogenase (EYD1), a gene encoding erythritol isomerase (EYI1) and a gene encoding erythritol isomerase (EYI2) of the genetically modified saprotroph is deleted.

Preferably, the at least one further inactivated gene selected from the group consisting of a gene encoding phospho-2-dehydro-3-deoxyheptonate aldolase 1 (Dhaps-1), a gene encoding erythritol utilization factor (EUF), a gene encoding erythrulose kinase (EYK1), a gene encoding erythritol dehydrogenase (EYD1), a gene encoding erythritol isomerase (EYI1) and a gene encoding erythritol isomerase (EYI2) of the genetically modified saprotroph is non-functional.

Particularly preferred, the at least one further inactivated gene selected from the group consisting of a gene encoding phospho-2-dehydro-3-deoxyheptonate aldolase 1 (Dhaps-1), a gene encoding erythritol utilization factor (EUF), a gene encoding erythrulose kinase (EYK1), a gene encoding erythritol dehydrogenase (EYD1), a gene encoding erythritol isomerase (EYI1) and a gene encoding erythritol isomerase (EYI2) of the genetically modified saprotroph is inactivated by gene knock-out, in particular gene replacement. Preferably, the at least one further inactivated gene selected from the group consisting of a gene encoding phospho-2-dehydro-3-deoxyheptonate aldolase 1 (Dhaps-1), a gene encoding erythritol utilization factor (EUF), a gene encoding erythrulose kinase (EYK1), a gene encoding erythritol dehydrogenase (EYD1), a gene encoding erythritol isomerase (EYI1) and a gene encoding erythritol isomerase (EYI2) of the genetically modified saprotroph is inactivated by gene replacement using a deletion cassette.

Preferably, the at least one further inactivated gene selected from the group consisting of a gene encoding phospho-2-dehydro-3-deoxyheptonate aldolase 1 (Dhaps-1), a gene encoding erythritol utilization factor (EUF), a gene encoding erythrulose kinase (EYK1), a gene encoding erythritol dehydrogenase (EYD1), a gene encoding erythritol isomerase (EYI1) and a gene encoding erythritol isomerase (EYI2) of the genetically modified saprotroph is inactivated, in particular made non-functional, by genome editing, in particular by meganucleases, zinc-finger nucleases (ZFNs), transcription activator-like effector nucleases (TALENs) or by the clustered regularly interspaced short palindromic repeats (CRISPR) system.

Preferably, the genetically modified saprotroph further comprises at least one gene encoding at least one transketolase. Preferably, the at least one gene encoding at least one transketolase is *tkl1,* in particular is codon-optimized *tkl1.* Preferably, the genetically modified saprotroph further comprises at least one gene encoding *tkl1* from *T. reesei,* in particular codon-optimized *tkl1* from *T. reesei.* Preferably, the at least one gene encoding at least one transketolase comprises the nucleotide sequence of SEQ ID No. 5, in particular consists of the nucleotide sequence of SEQ ID No. 5.

Further preferably, the at least one gene encoding at least one transketolase comprises a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 5. Preferably, the at least one gene encoding at least one transketolase consists of a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 5.

Preferably, the at least one transketolase comprises the amino acid sequence of SEQ ID No. 6, in particular consists of the amino acid sequence of SEQ ID No. 6.

According to another preferred option, the at least one transketolase comprises an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 6. Particularly preferred, the at least one transketolase consists of an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 6.

Further preferably, the at least one transketolase comprises an amino acid sequence as defined in SEQ ID No. 6, in which one, preferably two, preferably three, preferably five, preferably six, preferably seven, preferably eight, preferably nine, preferably ten, amino acids are exchanged by other naturally occurring amino acids. Particularly preferred, the at least one transketolase comprises an amino acid sequence as defined in SEQ ID No. 6, in which at most ten, preferably at most nine, preferably at most eight, preferably at most seven, preferably at most six, preferably at most five, preferably at most four, preferably at most three, preferably at most two, preferably at most one, amino acids are exchanged by other naturally occurring amino acids.

Preferably, the genetically modified saprotroph further comprises at least one gene encoding at least one transaldolase. Preferably, the at least one gene encoding at least one transaldolase is *tall,* in particular is codon-optimized *tall.* Preferably, the genetically modified saprotroph further comprises at least one gene encoding *tall* from *T. reesei,* in particular codon-optimized *tall* from *T. reesei.* Preferably, the at least one gene encoding at least one transaldolase comprises the nucleotide sequence of SEQ ID No. 7, in particular consist of the nucleotide sequence of SEQ ID No. 7.

Preferably, the at least one gene encoding at least one transaldolase comprises a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 7. Preferably, the at least one gene encoding at least one transaldolase consists of a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 7.

Preferably, the at least one transaldolase comprises the amino acid sequence of SEQ ID No. 8, in particular consists of the amino acid sequence of SEQ ID No. 8.

Particularly preferred, the at least one transaldolase comprises an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 8. Preferably, the at least one transaldolase consists of an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 8.

According to another preferred option, the at least one transaldolase comprises an amino acid sequence as defined in SEQ ID No. 8, in which one, preferably two, preferably three, preferably five, preferably six, preferably seven, preferably eight, preferably nine, preferably ten, amino acids are exchanged by other naturally occurring amino acids. Particularly preferred, the at least one transaldolase comprises an amino acid sequence as defined in SEQ ID No. 8, in which at most ten, preferably at most nine, preferably at most eight, preferably at most seven, preferably at most six, preferably at most five, preferably at most four, preferably at most three, preferably at most two, preferably at most one, amino acids are exchanged by other naturally occurring amino acids.

Further preferably, the genetically modified saprotroph further comprises at least one gene encoding at least one erythritol utilization factor (EUF). Preferably, the at least one gene encoding at least one erythritol utilization factor, is *euf1,* in particular is codon-optimized *euf1.* Preferably, the genetically modified saprotroph further comprises at least one gene encoding *euf1* from *T. reesei,* in particular codon-optimized *euf1* from *T. reesei.* Preferably, the at least one gene encoding at least one erythritol utilization factor (EUF) comprises the nucleotide sequence of SEQ ID No. 13, in particular consist of the nucleotide sequence of SEQ ID No. 13.

Preferably, the at least one gene encoding at least one erythritol utilization factor of the genetically modified saprotroph comprises a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 13. Preferably, the at least one gene encoding at least one erythritol utilization factor consists of a nucleotide sequence having at least 80%, preferably at least 85%, preferably at least 90%, preferably at least 95%, preferably at least 96%, preferably at least 97%, preferably at least 98%, preferably at least 99%, sequence identity to SEQ ID No. 13.

Preferably, the at least one erythritol utilization factor (EUF) comprises the amino acid sequence of SEQ ID No. 14, in particular consists of the amino acid sequence of SEQ ID No. 14.

Further preferably, the at least one erythritol utilization factor comprises an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 14. Preferably, the at least one erythritol utilization factor consists of an amino acid sequence having at least 80%, preferably at least 85%, preferably at least 90%, preferably at least 95%, preferably at least 96%, preferably at least 97%, preferably at least 98%, preferably at least 99%, sequence identity to SEQ ID No. 14.

Further preferably, the at least one erythritol utilization factor comprises an amino acid sequence as defined in SEQ ID No. 14, in which one, preferably two, preferably three, preferably five, preferably six, preferably seven, preferably eight, preferably nine, preferably ten, amino acids are exchanged by other naturally occurring amino acids. Particularly preferred, the at least one erythritol utilization factor comprises an amino acid sequence as defined in SEQ ID No. 14, in which at most ten, preferably at most nine, preferably at most eight, preferably at most seven, preferably at most six, preferably at most five, preferably at most four, preferably at most three, preferably at most two, preferably at most one, amino acids are exchanged by other naturally occurring amino acids.

According to a preferred option, the at least one gene encoding at least one membrane-bound alditol transporter, the at least one gene encoding at least one erythrose reductase, the at least one gene encoding at least one transketolase, the at least one gene encoding at least one transaldolase and/or the at least one gene encoding at least one erythritol utilization factor (EUF) of the genetically modified saprotroph is an exogenous polynucleotide sequence.

Preferably, the at least one gene encoding at least one membrane-bound alditol transporter, the at least one gene encoding at least one erythrose reductase, the at least one gene encoding at least one transketolase, the at least one gene encoding at least one transaldolase and/or the at least one gene encoding at least one erythritol utilization factor (EUF) of the genetically modified saprotroph is an endogenous polynucleotide sequence.

Further preferably, the genetically modified saprotroph comprises at least one exogenous gene encoding at least one membrane-bound alditol transporter. Preferably, genetically modified saprotroph comprises at least one endogenous gene encoding at least one membrane-bound alditol transporter.

Preferably, the genetically modified saprotroph comprises at least one exogenous gene encoding at least one erythrose reductase. According to another preferred option, the genetically modified saprotroph comprises at least one endogenous gene encoding at least one erythrose reductase.

Further preferred, the genetically modified saprotroph comprises at least one exogenous gene encoding at least one transketolase. In a preferred embodiment of the present invention, the genetically modified saprotroph comprises at least one endogenous gene encoding at least one transketolase.

Preferably, the genetically modified saprotroph comprises at least one exogenous gene encoding at least one transaldolase. Preferably, the genetically modified saprotroph comprises at least one endogenous gene encoding at least one transaldolase.

Preferably, the genetically modified saprotroph comprises at least one exogenous gene encoding at least one erythritol utilization factor (EUF). Preferably, the genetically modified saprotroph comprises at least one endogenous gene encoding at least one erythritol utilization factor (EUF).

According to a preferred option, the at least one gene encoding at least one membrane-bound alditol transporter, the at least one gene encoding at least one erythrose reductase, the at least one gene encoding at least one transketolase, the at least one gene encoding at least one transaldolase and/or the at least one gene encoding at least one erythritol utilization factor (EUF) is stably or transiently introduced into the genome of the genetically modified saprotroph.

Particularly preferred, the at least one gene encoding at least one membrane-bound alditol transporter, the at least one gene encoding at least one erythrose reductase, the at least one gene encoding at least one transketolase, the at least one gene encoding at least one transaldolase and/or the at least one gene encoding at least one erythritol utilization factor (EUF), is overexpressed.

Preferably, the at least one gene encoding at least one membrane-bound alditol transporter, in particular the at least one exogenous or endogenous gene encoding at least one membrane-bound alditol transporter, of the genetically modified saprotroph is overexpressed. Further preferred, the at least one gene encoding at least one erythrose reductase, in particular the at least one exogenous or endogenous gene encoding at least one erythrose reductase, of the genetically modified saprotroph is overexpressed. Preferably, the at least one gene encoding at least one transketolase, in particular the at least one exogenous or endogenous gene encoding at least one transketolase, of the genetically modified saprotroph is overexpressed. Further preferred, the at least one gene encoding at least one transaldolase, in particular the at least one exogenous or endogenous gene encoding at least one transaldolase, of the genetically modified saprotroph is overexpressed. Preferably, the at least one gene encoding at least one erythritol utilization factor (EUF), in particular the at least one exogenous or endogenous gene encoding at least one erythritol utilization factor (EUF), of the genetically modified saprotroph is overexpressed.

According to a further preferred option, the at least one gene encoding at least one membrane-bound alditol transporter, the at least one gene encoding at least one erythrose reductase, the at least one gene encoding at least one transketolase, the at least one gene encoding at least one transaldolase and/or the at least one gene encoding at least one erythritol utilization factor (EUF) of the genetically modified saprotroph is under the control of a constitutive or inducible promoter. Preferably, the constitutive or inducible promoter is a naturally occurring promoter. In a further preferred embodiment, the constitutive or inducible promoter is a synthetic promoter. Preferably, the constitutive promoter is selected from *pki, tef, gpd.* The inducible promoter is preferably selected from *bga1, bxl1, cbh1, cbh2, xyn1, xyn2.*

Preferably, the at least one gene of the genetically modified saprotroph encoding at least one membrane-bound alditol transporter is under the control of a promoter selected from *pki, tef, gpd,* preferably under control of a *pki* promoter. Preferably, the at least one gene of the genetically modified saprotroph encoding at least one erythrose reductase is under the control of a promoter selected from *pki, tef, gpd,* preferably under control of a *tef* promoter. Preferably, the at least one gene of the genetically modified saprotroph encoding at least one transketolase is under the control of a promoter selected from *pki, tef, gpd,* preferably under control of a *tef* promoter. Preferably, the at least one gene of the genetically modified saprotroph encoding at least one transaldolase is under the control of a promoter selected from *pki, tef, gpd,* preferably under control of a *tef* promoter. Preferably, the at least one gene of the genetically modified saprotroph encoding at least one erythritol utilization factor (EUF) is under the control of a promoter selected from *pki, tef, gpd,* preferably under control of a *tef* promoter.

According to a further preferred option, the at least one gene of the genetically modified saprotroph encoding at least one membrane-bound alditol transporter, the at least one gene of the genetically modified saprotroph encoding at least one erythrose reductase, the at least one gene of the genetically modified saprotroph encoding at least one transketolase, the at least one gene of the genetically modified saprotroph encoding at least one transaldolase and/or the at least one gene of the genetically modified saprotroph encoding at least one erythritol utilization factor (EUF) is present on a plasmid. Preferably, each of the at least one gene of the genetically modified saprotroph encoding at least one membrane-bound alditol transporter, the at least one gene of the genetically modified saprotroph encoding at least one erythrose reductase, the at least one gene of the genetically modified saprotroph encoding at least one transketolase, the at least one gene of the genetically modified saprotroph encoding at least one transaldolase and/or the at least one gene of the genetically modified saprotroph encoding at least one erythritol utilization factor (EUF) is present on a separate plasmid.

Particularly preferred, the genetically modified saprotroph is the *Trichoderma reesei* strain deposited at the Westerdijk Fungal Biodiversity Institute under CBS number 146708.

According to a preferred option, the at least one saprotroph is cultivated in step a) in a volume of at least 5 L, preferably at least 10 L, preferably, at least 25 L, preferably at least 50 L, preferably at least 100 L, preferably at least 250 L, preferably at least 500 L, preferably at least 1.000 L, preferably at least 2.500 L, preferably at least 7.500 L, preferably at least 10.000 L, preferably at least 25.000 L, preferably at least 50.000 L, preferably at least 75.000 L, preferably at least 100.000 L.

In a further preferred embodiment of the present invention, the at least one saprotroph is cultivated in step a) in the culture medium until the culture medium contains erythritol in a concentration of at least 250 mg/L, preferably at least 300 mg/L, preferably at least 350 mg/L, preferably at least 400 mg/L, preferably at least 450 mg/L, preferably at least 500 mg/L, preferably at least 600 mg/L, preferably at least 700 mg/L, preferably at least 800 mg/L, preferably at least 900 mg/L, preferably at least 1 g/L, preferably at least 2 g/L, preferably at least 3 g/L, preferably at least 4 g/L, preferably at least 5 g/L, preferably at least 6 g/L, preferably at least 7 g/L, preferably at least 8 g/L, preferably at least 9 g/L, preferably at least 10 g/L, preferably at least 15 g/L, preferably at least 20 g/L, preferably at least 25 g/L, preferably at least 30 g/L, preferably at least 35 g/L, preferably at least 40 g/L.

Particularly preferred, the at least one saprotroph is cultivated in the culture medium until the culture medium contains erythritol in the concentration of 250 mg/L to 40 g/L, preferably 500 mg/L to 40 g/L, preferably 750 mg/L to 40 g/L, preferably 1 g/L to 40 g/L.

According to a further preferred option, the at least one saprotroph is cultivated in the culture medium until the culture medium contains erythritol in the concentration of 5 g/L to 40 g/L, preferably 10 g/L to 40 g/L, preferably 15 g/L to 40 g/L, preferably 20 g/L to 40 g/L, preferably 25 g/L to 40 g/L, preferably 30 g/L to 40 g//L.

In a particularly preferred embodiment of the present invention, the at least one saprotroph is cultivated in step a) in the culture medium at a pH in the range of 2 to 7, preferably 2.5 to 6.5, preferably 2.5 to 6, preferably 3 to 5.5. Particularly preferred the at least one saprotroph is cultivated in the culture medium at a pH of at most 7, preferably at most 6.5, preferably at most 6, preferably at most 5.5, preferably at most 5.

According to a preferred option, the at least one saprotroph is cultivated in step a) in the culture medium at a temperature of 25 to 35 °C, preferably 26 to 34 °C, preferably 27 to 33 °C, preferably 28 to 32 °C, preferably 29 to 31 °C, preferably 30 °C.

Preferably, the at least one saprotroph is cultivated in step a) in the culture medium at a temperature of at least 15 °C, preferably at least 20 °C, preferably at least 25 °C, preferably at least 26 °C, preferably at least 27 °C, preferably at least 28 °C.

According to a further preferred option, the at least one saprotroph is cultivated in step a) in the culture medium at a temperature of at most 35 °C, preferably at most 34 °C, preferably at most 33 °C, preferably at most 32 °C, preferably at most 31 °C.

Preferably, the at least one saprotroph is cultivated in the culture medium in step a) for at least 24 hours, preferably at least 36 hours, preferably at least 48 hours, preferably at least 60 hours, preferably at least 72 hours, preferably at least 84 hours, preferably at least 96 hours, preferably at least 108 hours, preferably at least 120 hours, preferably at least 132 hours, preferably at least 144 hours, preferably at least 156 hours, preferably at least 168 hours.

According to a further preferred option, the at least one saprotroph is cultivated in the culture medium in step a) for at most 240 hours, preferably at most 216 hours, preferably at most 192 hours, preferably at most 180 hours, preferably at most 168 hours, preferably at most 156 hours, preferably at most 144 hours, preferably at most 132 hours, preferably at most 120 hours, preferably at most 108 hours, preferably at most 96 hours, preferably at most 84 hours, preferably at most 72 hours.

According to a preferred option, the cultivation of the at least one saprotroph in step a) is conducted without the addition of osmotically effective agents during cultivation, in particular without the addition of at least one salt during cultivation. Particularly preferred, the cultivation of the at least one saprotroph in step a) is conducted without the addition of sodium chloride (NaCl) during cultivation.

Preferably, the cultivation of the at least one saprotroph in step a) is conducted in batch-mode. Preferably, the cultivation of the at least one saprotroph in step a) is conducted in fed-batch mode. In another preferred embodiment, cultivation of the at least one saprotroph in step a) is conducted in continuous mode.

In a further preferred embodiment of the present invention the erythritol is recovered in step c) by crystallisation.

In a particularly preferred embodiment of the present invention, the recovery of erythritol in step b) comprises the steps:
i) removal of biomass, preferably removal of biomass by centrifugation and membrane filtration,
ii) decolorisation of the culture medium, preferably decolorisation of the culture medium with active carbon,
iii) desalting the culture medium, preferably desalting and decolorizing the culture medium by electrodialysis,
iv) preparative chromatography, preferably reverse phase chromatography using ion exclusion and size exclusion mechanisms, and
v) concentrating and crystalizing the erythritol.

According to a preferred embodiment of the present invention, a heat denaturing step is conducted prior to, during or after step i). Preferably, a heat denaturing step is conducted prior to step i). In a further preferred embodiment, a heat denaturing step is conducted during step i). Preferably, a heat denaturing step is conducted after step i).

Particularly preferred, the heat denaturing step includes heating the culture medium to a temperature of at least 45 °C, preferably at least 50 °C, preferably at least 55 °C, preferably at least 60 °C, preferably at least 65 °C, preferably at least 70 °C, preferably 75 °C, preferably at least 80 °C, preferably at least 85 °C, preferably at least 90 °C, preferably at least 95 °C.

According to a preferred option, the culture medium is subjected to heat denaturation for at least 10 seconds, preferably at least 20 seconds, preferably at least 30 seconds, preferably at least 40 seconds, preferably at least 50 seconds, preferably at least 1 min, preferably at least 2 min, preferably at least 3 min, preferably at least 4 min, preferably at least 5 min, preferably at least 6 min, preferably at least 7 min, preferably at least 8 min, preferably at least 9 min, preferably at least 10 min.

The heat denaturing step primarily serves to heat-inactivate proteins and enzymes in the culture medium before conducting further recovery steps.

According to a particularly preferred option, the erythritol recovered in step b), in particular the erythritol obtained in step v), has a purity of at least 90%, preferably at least 92%, preferably at least 94%, preferably at least 96%, preferably at least 97%, preferably at least 97,5%, preferably at least 98%, preferably at least 98,5%, preferably at least 99%, preferably at least 99,5%.

The term "genetically modified" and its grammatical equivalents as used herein refer to one or more alterations of a nucleic acid, e.g. the nucleic acid within the genome of an organism, in particular within the genome of a saprotroph. A "genetically modified" saprotroph can refer to a saprotroph with an added, deleted and/or altered, in particular inactivated, gene.

In the context of the present invention, the term "erythrose reductase" pertains to any enzyme that catalyses reversibly the reduction of an aldose to an alditol, wherein the enzyme has predominant specific activity for the reduction of erythrose to erythritol. Thus, an "erythrose reductase" according to the present invention exhibits a high specificity and affinity for the substrate erythrose. Particularly, the "erythrose reductase" has a higher specificity for erythrose than for any other substrate, such as glycerol.

The term "membrane-bound alditol transporter" as used herein designates a membrane protein suitable to reversibly transport alditols across the outer membrane, in particular to transport intracellularly produced alditols to the culture medium. According to the present invention, a "membrane-bound alditol transporter" is suitable to transport intracellularly produced erythritol across the membrane to the culture medium.

In the context of the present invention, the term "transketolase" refers to an enzyme of the pentose phosphate pathway catalysing the thiamine-dependent reversible transfer of a C-2 unit from a ketose donor to an acceptor aldopentose. In particular, the "transketolase" catalyses the transfer of a C-2 unit from xylulose-5-phosphate to ribose-5-phosphate forming seduheptulose-7-phosphate and glyceraldehyde-3-phosphate.

The term "transaldolase" designates an enzyme of the non-oxidative branch of the pentose phosphate pathway catalysing the reversible transfer of a C-3 unit from a ketose donor to an acceptor aldose. In particular, the "transketolase" catalyses the transfer of a C-3 unit from sedoheptulose-7-phosphate to glyceraldehyde-3-phosphate forming erythrose-4-phosphate and fructose-6-phosphate.

In the context of the present invention, an "inactivated" gene is a gene which can temporarily or permanently not be transcribed or can be transcribed but the transcript is not accessible for gene translation. According to the present invention, the inactivation of a gene includes but is not limited to inactivation by partial or complete gene deletion, partial or complete gene replacement, gene repression, gene insertion, and gene mutation. "Inactivation" in the context of the present invention further includes any post-transcriptional gene regulation, in particular by RNAi or siRNA, resulting in inhibition of translation of the transcript into the gene product.

In the context of the present invention, a "non-functional" gene is a gene that is present in the genome of an organism or a plasmid in an organism which gene is either not transcribed or is transcribed but the transcript is not translated to the gene product.

In the context of the present invention, a "deleted" gene is a gene that has previously been present in the genome of an organism or a plasmid in an organism but has been completely removed from the genome of the organism or the plasmid in the organism.

In the context of the present invention, the verb "to overexpress" refers to the artificial expression of a gene in increased quantity. This includes the inducible overexpression of a gene which can be regulated using an inducible promoter and the constant overexpression of a gene using a constitutive promoter.

The term "oligomeric sugars" or "oligomeric C-5 and C-6 sugars" is used herein for di-, oligo-, and polysaccharides which are composed of at least two monomers, preferably composed of at least two monomers selected from C-5 and C-6 sugars.

In the context of the present invention, the term "a" is meant to include the meaning of "one" or "one or more".

In the context of the present invention, the term "comprising" preferably has the meaning of "containing" or "including", meaning including the specifically identified elements without excluding the presence of further elements. However, in a preferred embodiment, the term "comprising" is also understood to have the meaning of "consisting essentially of" and in a further preferred embodiment the meaning of "consisting of".

The terms "and/or" is used herein to express the disclosure of any combination of individual elements connected within a listing. Solely for illustrative purposes, the expression "A, B, and/or C" can mean A individually, B individually, C individually, (A and B), (B and C), (A and C), and (A, B and C).

Further preferred embodiments of the invention are subject of the subclaims.

The invention is further described by way of the following example and accompanying figures.
**Figure 1** shows the influence of the carbon source concentration in the culture medium on erythritol production by *Trichoderma reesei* after 168 hours incubation in a culture medium comprising either 50 g/L, 70 g/L or 90 g/L glucose as carbon source and 80 mM urea as nitrogen source.
**Figure 2** shows the influence of the nitrogen source concentration in the culture medium on erythritol production by *Trichoderma reesei* after 168 hours incubation in a culture medium comprising 70 g/L glucose as carbon source and either 20 mM, 60 mM or 80 mM urea as nitrogen source.

### Example:

### 1. Biotechnological production of erythritol

### 1.1 Influence of the carbon source concentration on erythritol production

*Trichoderma reesei* was cultivated in 250 mL flasks each comprising 100 mL culture medium containing either 50 g/L, 70 g/L or 90 g/L glucose as carbon source and 80 mM urea as nitrogen source.

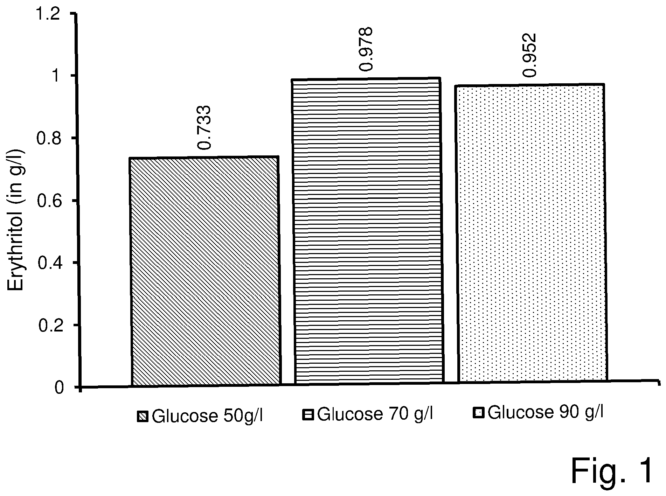

After 168 hours of cultivation, the erythritol concentration was measured in the culture medium of the samples. The average concentration of erythritol in the samples was 0.733 g/L (50 g/L glucose), 0.978 g/L (70 g/L glucose), and 0.952 g/L (90 g/L glucose) (see **Fig. 1****).**

### 1.2 Influence of the nitrogen source concentration on erythritol production

In a similar experiment, *Trichoderma reesei* was cultivated in 250 mL flasks each comprising 100 mL culture medium containing 70 g/L glucose as carbon source and either 20 mM, 60 mM or 80 mM urea as nitrogen source.

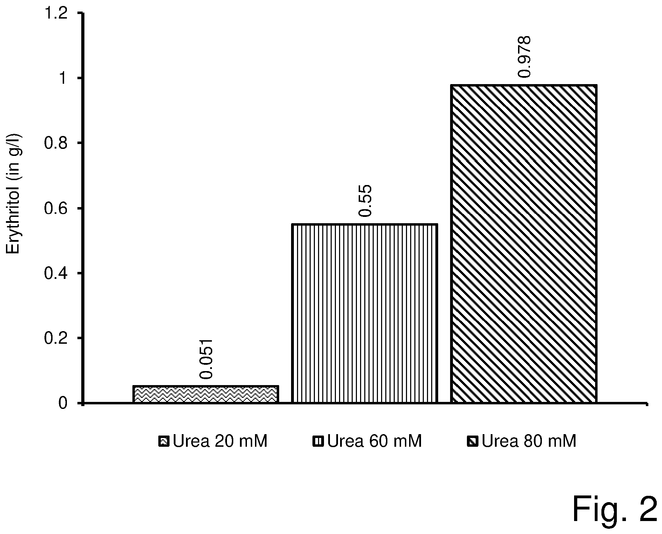

After 168 hours of cultivation, the erythritol concentration in the culture medium of the samples was measured. The average concentration of erythritol in the samples was 0.051 g/L (20 mM urea), 0.55 g/L (60 mM urea), and 0.978 g/L (80 mM urea) (see **Fig. 2****).**

## Claims

1. A method for the production of erythritol, comprising the steps:
a) cultivating at least one saprotroph selected from the genera *Hypocrea* and *Trichoderma* in a culture medium comprising a carbon source in a concentration of 60 to 100 g/L and a nitrogen source, so as to obtain erythritol in the culture medium, and
b) recovering erythritol from the culture medium,
wherein the carbon source is a monomeric or oligomeric C-6 sugar or a mixture thereof, wherein the C-6 sugar is selected from the group consisting of glucose and fructose.

2. The method according to claim 1, wherein the culture medium comprises the nitrogen source in a concentration of at least 50 mM, preferably at least 60 mM, preferably at least 70 mM.

3. The method according to any one of the preceding claims, wherein the culture medium comprises a lignocellulose comprising hydrolysate.

4. The method according to any one of the preceding claims, wherein the carbon source is glucose.

5. The method according to any one of claims 1 to 3, wherein the carbon source comprises a hydrolysate obtained by hydrothermal treatment of a cellulose- and/or starch-comprising raw material.

6. The method according to any one of the preceding claims, wherein the nitrogen source is urea.

7. The method according to any one of the preceding claims, wherein the saprotroph is *Hypocrea jecorina (Trichoderma reesei)*

8. The method according to any one of the preceding claims, wherein in step a) the at least one saprotroph is cultivated in the culture medium until the culture medium contains erythritol in a concentration of at least 250 mg/L, preferably at least 500 mg/L, preferably at least 1 g/L.

9. The method according to any one of the preceding claims, wherein in step a) the at least one saprotroph is cultivated in the culture medium at a pH in the range of 2 to 7, preferably 3 to 5.5.

10. The method according to any one of the preceding claims, wherein the erythritol is recovered in step b) by crystallisation.

11. The method according to any one of the preceding claims, wherein the recovery of erythritol in step b) comprises the steps:
i) removal of biomass, preferably removal of biomass by centrifugation and membrane filtration,
ii) decolorisation of the culture medium, preferably decolorisation of the culture medium with active carbon,
iii) desalting the culture medium, preferably desalting and decolorizing the culture medium by electrodialysis,
iv) preparative chromatography, preferably reverse phase chromatography using ion exclusion and size exclusion mechanisms, and
v) concentrating and crystallising the erythritol.

12. The method according to claim 11, wherein a heat denaturing step is conducted prior to, during or after step i).

## Patentansprüche

1. Verfahren zur Herstellung von Erythritol, umfassend die Schritte:
a) Kultivieren mindestens eines Saprotrophs, ausgewählt aus den Gattungen *Hypocrea* und *Trichoderma,* in einem Kulturmedium umfassend eine Kohlenstoffquelle in einer Konzentration von 60 bis 100 g/L und eine Stickstoffquelle, um Erythritol im Kulturmedium zu erhalten, und
b) Gewinnen von Erythritol aus dem Kulturmedium,
wobei die Kohlenstoffquelle ein monomerer oder oligomerer C-6-Zucker oder ein Gemisch davon ist, wobei der C-6-Zucker ausgewählt ist aus der Gruppe bestehend aus Glucose und Fructose.

2. Verfahren gemäß Anspruch 1, wobei das Kulturmedium die Stickstoffquelle in einer Konzentration von mindestens 50 mM, bevorzugt mindestens 60 mM, bevorzugt mindestens 70 mM, umfasst.

3. Verfahren gemäß einem der vorhergehenden Ansprüche, wobei das Kulturmedium ein Lignocellulose-umfassendes Hydrolysat umfasst.

4. Verfahren gemäß einem der vorhergehenden Ansprüche, wobei die Kohlenstoffquelle Glucose ist.

5. Verfahren gemäß einem der Ansprüche 1 bis 3, wobei die Kohlenstoffquelle ein Hydrolysat umfasst, das durch hydrothermale Behandlung eines Cellulose- und/oder Stärke-umfassenden Rohmaterials erhalten wurde.

6. Verfahren gemäß einem der vorhergehenden Ansprüche, wobei die Stickstoffquelle Harnstoff ist.

7. Verfahren gemäß einem der vorhergehenden Ansprüche, wobei der Saprotroph *Hypocrea jecorina* (*Trichoderma reesei*) ist.

8. Verfahren gemäß einem der vorhergehenden Ansprüche, wobei in Schritt a) der mindestens eine Saprotroph im Kulturmedium kultiviert wird, bis das Kulturmedium Erythritol in einer Konzentration von mindestens 250 mg/L, bevorzugt mindestens 500 mg/L, bevorzugt mindestens 1 g/L, enthält.

9. Verfahren gemäß einem der vorhergehenden Ansprüche, wobei in Schritt a) der mindestens eine Saprotroph in dem Kulturmedium bei einem pH-Wert im Bereich von 2 bis 7, bevorzugt 3 bis 5,5, kultiviert wird.

10. Verfahren gemäß einem der vorhergehenden Ansprüche, wobei das Erythritol in Schritt b) durch Kristallisation gewonnen wird.

11. Verfahren gemäß einem der vorhergehenden Ansprüche, wobei die Gewinnung von Erythritol in Schritt b) die Schritte umfasst:
i) Entfernen der Biomasse, bevorzugt Entfernen der Biomasse durch Zentrifugation und Membranfiltration,
ii) Entfärbung des Kulturmediums, bevorzugt Entfärbung des Kulturmediums mit Aktivkohle,
iii) Entsalzen des Kulturmediums, bevorzugt Entsalzen und Entfärben des Kulturmediums durch Elektrodialyse,
iv) präparative Chromatographie, bevorzugt Umkehrphasenchromatographie unter Verwendung von Ionenausschluss- und Größenausschlussmechanismen, und
v) Konzentrieren und Kristallisieren des Erythritols.

12. Verfahren gemäß Anspruch 11, wobei vor, während oder nach Schritt i) ein Hitze-Denaturierungsschritt durchgeführt wird.

## Revendications

1. Un procédé de production d'érythritol, comprenant les étapes :
a) cultiver au moins un saprotrophe choisi parmi les genres *Hypocrea* et *Trichoderma* dans un milieu de culture comprenant une source de carbone à une concentration de 60 à 100 g/L et une source d'azote, de manière à obtenir de l'érythritol dans le milieu de culture, et
b) obtenir l'érythritol du milieu de culture,
dans lequel la source de carbone est un sucre C-6 monomère ou oligomère ou un mélange de ceux-ci, dans lequel le sucre C-6 est choisi dans le groupe constitué par le glucose et le fructose.

2. Le procédé selon la revendication 1, dans lequel le milieu de culture comprend la source d'azote à une concentration d'au moins 50 mM, préférablement d'au moins 60 mM, préférablement d'au moins 70 mM.

3. Le procédé selon l'une quelconque des revendications précédentes, dans lequel le milieu de culture comprend un hydrolysat comprenant de la lignocellulose.

4. Le procédé selon l'une quelconque des revendications précédentes, dans lequel la source de carbone est le glucose.

5. Le procédé selon l'une quelconque des revendications 1 à 3, dans lequel la source de carbone comprend un hydrolysat obtenu par traitement hydrothermique d'une matière première comprenant de la cellulose et/ou de l'amidon.

6. Le procédé selon l'une quelconque des revendications précédentes, dans lequel la source d'azote est l'urée.

7. Le procédé selon l'une quelconque des revendications précédentes, dans lequel le saprotrophe est *Hypocrea jecorina* (*Trichoderma reesei*).

8. Le procédé selon l'une quelconque des revendications précédentes, dans lequel, à l'étape a), le au moins un saprotrophe est cultivé dans le milieu de culture jusqu'à ce que le milieu de culture contienne de l'érythritol à une concentration d'au moins 250 mg/L, préférablement d'au moins 500 mg/L, préférablement d'au moins 1 g/L.

9. Le procédé selon l'une quelconque des revendications précédentes, dans lequel, à l'étape a), le au moins un saprotrophe est cultivé dans le milieu de culture à un pH dans la plage de 2 à 7, préférablement de 3 à 5,5.

10. Le procédé selon l'une quelconque des revendications précédentes, dans lequel l'érythritol est obtenu à l'étape b) par cristallisation.

11. Le procédé selon l'une quelconque des revendications précédentes, dans lequel la abattage de l'érythritol dans l'étape b) comprend les étapes :
i) enlèvement de la biomasse, préférablement enlèvement de la biomasse par centrifugation et filtration sur membrane,
ii) décoloration du milieu de culture, préférablement décoloration du milieu de culture avec du charbon actif,
iii) dessalage du milieu de culture, préférablement dessalage et décolorer du milieu de culture par électrodialyse,
iv) chromatographie préparative, préférablement chromatographie en phase inverse utilisant des mécanismes d'exclusion ionique et d'exclusion de taille, et
v) concentrer et cristallisation de l'érythritol.

12. Le procédé selon la revendication 11, dans lequel une étape de dénaturation thermique est effectuée avant, pendant ou après l'étape i).
